# EUROPEAN PATENT SPECIFICATION

(11) **EP 3 554 573 B1**
(45) Date of publication and mention of the grant of the patent: **30.04.2025**
(21) Application number: 17818494.1
(22) Date of filing: 11.12.2017
(51) Int. Cl.: A61M 1/00, A61F 13/05

(54) **NEGATIVE PRESSURE WOUND CLOSURE DEVICE**
UNTERDRUCKWUNDVERSCHLUSSVORRICHTUNG
DISPOSITIF DE FERMETURE DE PLAIE PAR PRESSION NÉGATIVE

(30) Priority: 16.12.2016 US 201662435553 P; 19.04.2017 US 201762487410 P
(43) Date of publication of application: 23.10.2019
(73) Proprietor: Smith & Nephew PLC, Watford Hertfordshire WD18 8YE (GB)
(72) Inventor: DELLA-PORTA, Louis, Elslack, Skipton, BD23 3BB (GB)
(74) Representative: Guy, Mark Robert
(86) International application number: PCT/EP2017/082169
(87) International publication number: WO 2018/108785

(56) References cited:
- WO-A1-2014/014871
- WO-A1-2016/176513
- US-A1- 2005 142 331
- US-A1- 2015 320 602

## Description

### BACKGROUND

### Field of the Invention

This application describes embodiments of apparatuses, methods, and systems for the treatment of wounds, specifically to aid in the closure of large wounds, in conjunction with the administration of negative pressure.

### Description of the Related Art

Negative pressure wound therapy has been used in the treatment of wounds, and in many cases can improve the rate of healing while also removing exudates and other deleterious substances from the wound site.

Abdominal compartment syndrome is caused by fluid accumulation in the peritoneal space due to edema and other such causes, and results in greatly increased intra-abdominal pressure that may cause organ failure eventually resulting in death. Causes may include sepsis or severe trauma. Treatment of abdominal compartment syndrome may require an abdominal incision to permit decompression of the abdominal space, and as such, a large wound may be created onto the patient. Closure of this wound, while minimizing the risk of secondary infections and other complications, and after the underlying edema has subsided, then becomes a priority. However, acute open abdominal conditions may be caused by other reasons in addition to compartment syndrome, as described further below.

Other large or incisional wounds, either as a result of surgery, trauma, or other conditions, may also require closure. For example, wounds resulting from stemiotomies, fasciotomies, and other abdominal wounds may require closure. Wound dehiscence of existing wounds is another complication that may arise, possibly due to incomplete underlying fascial closure, or secondary factors such as infection.

Existing negative pressure treatment systems, while permitting eventual wound closure, still require lengthy closure times. Although these may be combined with other tissue securement means, such as sutures, there is also a risk that underlying muscular and fascial tissue is not appropriately reapproximated so as to permit complete wound closure. Further, when foam or other wound fillers are inserted into the wound, the application of negative pressure to the wound and the foam may cause atmospheric pressure to bear down onto the wound, compressing the foam downward and outward against the margins of the wound. This downward compression of the wound filler slows the healing process and slows or prevents the joining of wound margins. Additionally, inflammation of the fascia in the form of certain types of fasciitis can lead to rapid and excessive tissue loss, potentially meriting the need for more advanced negative pressure treatment systems. Accordingly, there is a need to provide for an improved apparatus, method, and system for the treatment and closure of wounds. Relevant prior art is disclosed in WO 2014/014871 A1, US 2015/320602 A1, WO 2016/176513 A1, US 2005/142331 A1.

### SUMMARY

The invention is defined in the appended claims.

Embodiments of the present invention relate to negative pressure wound closure devices, methods, and systems that facilitate closure of a wound. It will be understood by one of skill in the art that the wounds described herein this specification may encompass any wound, and are not limited to a particular location or type of wound. The devices, methods, and systems may operate to reduce the need for repetitive replacement of wound filler material currently employed and can advance the rate of healing. The devices, methods (not claimed), and systems are simultaneously used with negative pressure to remove wound fluids.

In certain (not claimed) embodiments, an apparatus for treating a wound with negative pressure wound therapy is provided. The apparatus comprises a stabilizing structure for insertion into a wound. The stabilizing structure comprises a length corresponding to a y-direction and extending along a central longitudinal axis of the stabilizing structure between a first end and a second end of the stabilizing structure, a width corresponding to an x-direction, the width being transverse to the length and extending along a central transverse axis of the stabilizing structure between a first side and a second side of the stabilizing structure, and a height corresponding to a z-direction, the height being transverse to the length and the width and extending between a top surface and a bottom surface of the stabilizing structure. The length and width of the stabilizing structure may each be greater than the height. The stabilizing structure may further comprise a plurality of cells defined by one or more walls, the cells being provided side-by-side in a horizontal plane parallel to the x-direction and the y-direction, wherein each of the cells has a top end and a bottom end with an opening extending through the top and bottom ends in the z-direction. The stabilizing structure may also be configured such that upon application of negative pressure to the wound when the stabilizing structure is inserted into the wound, the stabilizing structure collapses more in the horizontal plane than in the z-direction, and the stabilizing structure collapses more in the x-direction than in the y-direction.

In certain (not claimed) embodiments, an apparatus for treating a wound with negative pressure wound therapy is provided. The apparatus comprises a stabilizing structure for insertion into a wound. The stabilizing structure comprises a length corresponding to a y-direction and extending along a central longitudinal axis of the stabilizing structure between a first end and a second end of the stabilizing structure, a width corresponding to an x-direction, the width being transverse to the length and extending along a central transverse axis of the stabilizing structure between a first side and a second side of the stabilizing structure, and a height corresponding to a z-direction, the height being transverse to the length and the width and extending between a top surface and a bottom surface of the stabilizing structure. The length and width of the stabilizing structure may each be greater than the height. The stabilizing structure may further comprise a plurality of cells defined by one or more walls, the cells being provided side-by-side in a horizontal plane parallel to the x-direction and the y-direction, wherein each of the cells has a top end and a bottom end with an opening extending through the top and bottom ends in the z-direction. The cells may be provided in a plurality of rows extending width-wise across the stabilizing structure, and wherein cells of adjacent rows are staggered relative to each other. The stabilizing structure may also be configured such that upon application of negative pressure to the wound when the stabilizing structure is inserted into the wound, the stabilizing structure collapses more in the horizontal plane than in the z-direction, and the stabilizing structure collapses more in the x-direction than in the y-direction.

In certain (not claimed) embodiments, an apparatus for treating a wound with negative pressure wound therapy is provided. The apparatus comprises a stabilizing structure for insertion into a wound. The stabilizing structure comprises a length corresponding to a y-direction and extending along a central longitudinal axis of the stabilizing structure between a first end and a second end of the stabilizing structure, a width corresponding to an x-direction, the width being transverse to the length and extending along a central transverse axis of the stabilizing structure between a first side and a second side of the stabilizing structure, and a height corresponding to a z-direction, the height being transverse to the length and the width and extending between a top surface and a bottom surface of the stabilizing structure. The length and width of the stabilizing structure may each be greater than the height. The stabilizing structure may further comprise a plurality of cells defined by one or more walls, the cells being provided side-by-side in a horizontal plane parallel to the x-direction and the y-direction, wherein each of the cells has a top end and a bottom end with an opening extending through the top and bottom ends in the z-direction. At least one of the cells may have a hexagon shape. The stabilizing structure may also be configured such that upon application of negative pressure to the wound when the stabilizing structure is inserted into the wound, the stabilizing structure collapses more in the horizontal plane than in the z-direction, and the stabilizing structure collapses more in the x-direction than in the y-direction.

In the claimed embodiment, an apparatus for treating a wound with negative pressure wound therapy is provided. The apparatus comprises a stabilizing structure for insertion into a wound. The stabilizing structure comprises a length corresponding to a y-direction and extending along a central longitudinal axis of the stabilizing structure between a first end and a second end of the stabilizing structure, a width corresponding to an x-direction, the width being transverse to the length and extending along a central transverse axis of the stabilizing structure between a first side and a second side of the stabilizing structure, and a height corresponding to a z-direction, the height being transverse to the length and the width and extending between a top surface and a bottom surface of the stabilizing structure. The length and width of the stabilizing structure are each greater than the height. The stabilizing structure further comprises a plurality of cells defined by one or more walls, the cells being provided side-by-side in a horizontal plane parallel to the x-direction and the y-direction, wherein each of the cells has a top end and a bottom end with an opening extending through the top and bottom ends in the z-direction. The cells are provided in a plurality of rows extending width-wise across the stabilizing structure, and wherein cells of adjacent rows are staggered relative to each other. At least one of the cells has a concave-hexagon shape comprising two parallel sides and two internal angles greater than 180 degrees. The stabilizing structure is configured such that upon application of negative pressure to the wound when the stabilizing structure is inserted into the wound, the stabilizing structure collapses more in the horizontal plane than in the z-direction, and the stabilizing structure collapses more in the x-direction than in the y-direction.

In certain embodiments, at least one of the cells, or all of the cells, is or are defined by two straight walls aligned in parallel fashion along the y-direction and at least four side walls extending along the x-direction wherein the side walls connect the two straight walls. In some embodiments, at least two side walls meet to form an inner angle greater than 180 degrees. In other embodiments, at least two side walls meet to form an inner angle less than 180 degrees.

In certain embodiments, at least one of the cells, or all of the cells, has or have a hexagon shape. The hexagon shape is a concave-hexagon shape or (in a non-claimed embodiment) a convex-hexagon shape. The concave-hexagon shape has two parallel sides and two inner angles greater than 180 degrees. In other (not claimed) embodiments, the convex-hexagon shape has all inner angles less than 180 degrees.

In certain embodiments, at least one cell having a concave-hexagon shape may be surrounded by and shares a wall with six cells also having a concave-hexagon shape. In certain embodiments, the sum of lengths of said two straight walls is equal to or greater than the sum of lengths of said four side walls. In other embodiments, the convex-hexagon shape has all inner angles less than 180 degrees.

In certain embodiments, the stabilizing structure comprises at least one node where three or fewer walls meet. In certain embodiments, at all nodes three or fewer walls meet. In certain embodiments, the stabilizing structure comprises some or all of the cells with the same shape. In certain embodiments, the stabilizing structure comprises some or all of the cells with same size.

In certain embodiments, the cells are configured such that upon collapse, each cell shows zero or substantially no change in dimension in y-direction. In other embodiments, the cells are configured such that upon collapse, each cell increases in dimension in the y-direction.

In certain embodiments, the stabilizing structure is configured to allow portions of the device to separate from the remainder of the device. The stabilizing structure my comprise perforations or detachable sections that allow portions of the structure to separate from the remainder of structure. In certain embodiments, the stabilizing structure further comprises a wound wall liner that surrounds walls of the outermost cells.

### BRIEF DESCRIPTION OF THE DRAWINGS

Fig. 1 illustrates an embodiment of a negative pressure treatment system.
Fig. 2 illustrates a top view of an embodiment of a stabilizing structure inserted into a wound.
Fig. 3A illustrates a perspective view of an embodiment of a stabilizing structure having concave-hexagon cells.
Fig. 3B illustrates a top view of the embodiment of a stabilizing structure shown by Fig. 3A.
Figs. 4A-D illustrate schematic views of an embodiment of a stabilizing structure having concave-hexagon cells in a natural state and a collapsed state.
Fig. 5 illustrates a schematic view of an embodiment of a stabilizing structure having concave-hexagon cells.
Fig. 6 illustrates an embodiment (not claimed) of a stabilizing structure having convex-hexagon cells.
Figs. 7A-7B illustrate an embodiment (not claimed) of a stabilizing structure having convex-hexagon cells in a natural state and a collapsed state.
Fig. 8 illustrates an embodiment of a stabilizing structure illustrating detachable or removable portions.
Fig. 9 illustrates an embodiment of a stabilizing structure illustrating a portion of the structure removed.

### DETAILED DESCRIPTION

Embodiments disclosed in this section or elsewhere in this specification relate to apparatuses and methods of treating a wound with reduced pressure, including pump and wound dressing components and apparatuses. Certain embodiments of stabilizing structures and related apparatuses and methods of treating a wound with reduced pressure, including pump and wound dressing components and apparatuses, have been described in PCT App. No. PCT/US2013/050698, filed July 16, 2013 titled "Negative Pressure Wound Closure Device," published as WO 2014/014922 A1 which is to be considered a part of this specification. Specifically, WO 2014/014922 A1 describes a stabilizing structure for insertion into a wound, which is configured to aid in the closure of large wounds, in conjunction with the administration of negative pressure.

Further, examples of such applications where additional disclosure relating to the preceding descriptions may be found include U.S. Patent No. 8,235,955, titled "Wound treatment apparatus and method," issued August 7, 2012 and U.S. Patent No. 7,753,894, titled "Wound cleansing apparatus with stress," issued July 13, 2010. Other applications that may contain teachings relevant for use with the embodiments described in this section or elsewhere in this specification may include Application Serial No. 12/886,088, titled "Systems And Methods For Using Negative Pressure Wound Therapy To Manage Open Abdominal Wounds," filed September 20, 2010, published as US 2011/0213287; Application Serial No. 13/092,042, titled "Wound Dressing And Method Of Use," filed April 21, 2011, published as US 2011/0282309; and Application Serial No. 13/365,615, titled "Negative Pressure Wound Closure Device," filed February 3, 2012, published as US 2012/0209227. Still more applications that may contain teachings relevant for use with the embodiments described in this specification are Application Serial No. 13/942,493, titled "Negative Pressure Wound Closure Device," filed July 15, 2013, published as US 2014/0180225; PCT App. No. PCT/US2013/050619, filed July 16, 2013 titled "Negative Pressure Wound Closure Device," published as WO 2014/014871 A1; PCT App. No. PCT/IB2013/01555, titled "Devices and Methods for Treating and Closing Wounds with Negative Pressure," filed May 5, 2013, published as WO 2013/175309 A1; PCT App. No. PCT/US2014/025059, titled "Negative Pressure Wound Closure Device and Systems and Methods of Use in Treating Wounds with Negative Pressure," filed March 12, 2014, published as WO 2014/165275 A1; PCT App. No. PCT/GB2014/050746, "Compressible Wound Fillers and Systems and Methods of Use in Treating Wounds with Negative Pressure," filed Mar 13, 2014, published as WO 2014/140578 A1; and PCT/US2014/061627 "Negative Pressure Wound Closure Device," filed Oct 21, 2014, published as WO 2015/061352 A2.

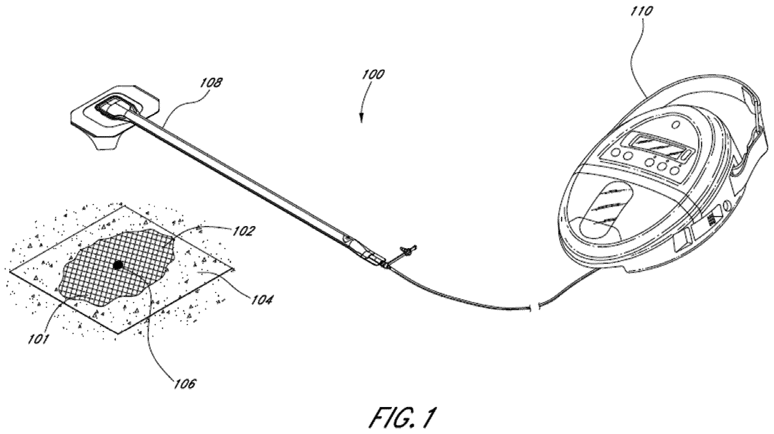

Figure 1 illustrates an embodiment of a negative pressure treatment system 100 that comprises a wound packer 102 inserted into a wound 101. The wound packer 102 may comprise porous materials such as foam, and in some embodiments may comprise one or more embodiments of wound closure devices or stabilizing structures described in further detail in this section or elsewhere in this specification. In some embodiments, the perimeter or top of any wound closure device inserted into the wound 101 may also be covered with foam or other porous materials. A single drape 104 or multiple drapes may be placed over the wound 101, and is preferably adhered or sealed to the skin on the periphery of the wound 101 so as to create a fluid-tight seal. An aperture 106 may be made through the drape 104 which can be manually made or preformed into the drape 104 so as to provide a fluidic connection from the wound 101 to a source of negative pressure such as a pump 110. Preferably, the fluidic connection between the aperture 106 and the pump 110 is made via a conduit 108. In some embodiments, the conduit 108 may comprise a RENASYS^{®} Soft Port^{™}, manufactured by Smith & Nephew. Of course, in some embodiments, the drape 104 may not necessarily comprise an aperture 106, and the fluidic connection to the pump 110 may be made by placing the conduit 108 below the drape. In some wounds, particularly larger wounds, multiple conduits 108 may be used, fluidically connected via one or more apertures 106.

In use, the wound 101 is prepared and cleaned. In some cases, such as abdominal wounds, a non- or minimally-adherent organ protection layer (not illustrated) may be applied over any exposed viscera. The wound packer 102 is then inserted into the wound, and is covered with the drape 104 so as to form a fluid-tight seal. A first end of the conduit 108 is then placed in fluidic communication with the wound, for example via the aperture 106. The second end of the conduit 108 is connected to the pump 110. The pump 110 may then be activated so as to supply negative pressure to the wound 101 and evacuate wound exudate from the wound 101. As will be described in additional detail below and in relation to the embodiments of the foregoing wound closure devices, negative pressure may also aid in promoting closure of the wound 101, for example by approximating opposing wound margins.

Any structure or component disclosed herein this section or elsewhere in the specification may comprise a radiopaque material. A radiopaque material advantageously allows a clinician to more easily find pieces of the wound closure device that may have come loose from the structure and become lost in the wound. Some examples of radiopaque materials include barium sulfate, bismuth trioxide, bismuth subcarbonate, bismuth oxychloride, and tungsten.

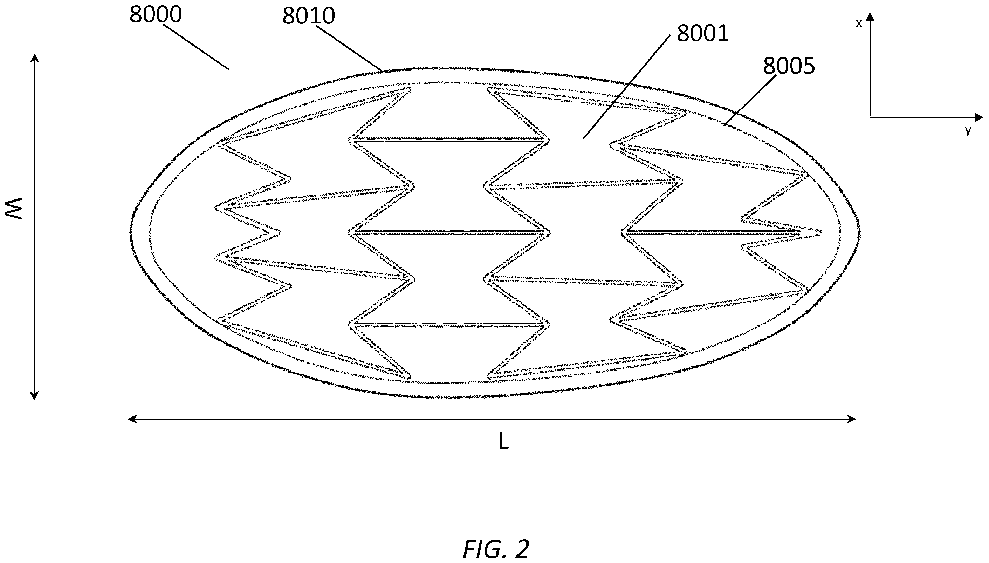

### Stabilizing Structures and Wound Closure Devices of Figure 2

Figure 2 illustrates an embodiment of a stabilizing structure 8000 inserted into a generally oval-shaped wound 8010 in a top view, comprising a plurality of cells 8001 arranged side-by-side. The stabilizing structure may have a longitudinal length that is aligned with a longitudinal axis of the wound, and a width that is perpendicular or transverse to the longitudinal axis. Although not illustrated in Figure 2, the stabilizing structure may have a height defined between a top surface and a bottom surface of the stabilizing structure, wherein the height is less than each of the length and width of the stabilizing structure. In some embodiments, the height may be uniform across the entire width and length of the stabilizing structure. In some embodiments, the top and bottom surfaces of the stabilizing structure may be planar.

As illustrated, each cell may be defined by one or more walls, each cell having a top end and a bottom end with an opening extending through the top and bottom ends. As with the other stabilizing structures described herein this section and elsewhere in the specification, the stabilizing structure 8000 is configured to collapse by collapsing one or more cells 8001. In some embodiments, the cells are all of the same approximate shape and size when in an uncollapsed configuration. However, in other embodiments, the cells may be of different shapes and sizes when in an uncollapsed configuration.

As used in this section or elsewhere in this specification, the x direction, when referring to the stabilizing structure, generally refers to a direction or plane generally parallel to the skin surrounding the wound. The y direction, when referring to the stabilizing structure, generally refers to a direction or plane generally parallel to the skin surrounding the wound and extending perpendicular to the x direction. The z direction, when referring to the stabilizing structure, generally refers to a direction or plane extending perpendicular to the x direction and the y direction. The term "width," when referring to a stabilizing structure, generally refers to a dimension of the stabilizing structure taken in the x direction along which the stabilizing structure is longest. The term "length," when referring to a stabilizing structure, generally refers to a dimension of the stabilizing structure taken in the y direction along which the stabilizing structure is longest. The term "height," when referring to a stabilizing structure, generally refers to a dimension of the stabilizing structure taken in the z direction along which the stabilizing structure is longest. The terms "width," "length," and "height" may also be used to describe the cells within the stabilizing structures and wound closure devices described throughout this specification. When describing these structures or devices, these terms should not be construed to require that the structures or devices necessarily be placed into a wound in a certain orientation, though in certain embodiments, it may be preferable to do so.

In some embodiments, the stabilizing structure 8000 can collapse in any manner described in this section or elsewhere in this specification with or without the application of negative pressure. For example, the stabilizing structure may collapse significantly more in one plane than in another plane upon application of negative pressure. In some embodiments, the stabilizing structure is configured to collapse more in a horizontal plane parallel to the length and width of the stabilizing structure than in a vertical plane perpendicular to the horizontal plane. In some embodiments, the stabilizing structure may collapse along the width of the stabilizing structure while remaining relatively rigid along the length of the stabilizing structure and in the vertical direction. In some embodiments, the stabilizing structure may collapse along the width of the stabilizing structure while extending along the length of the stabilizing structure and remaining relatively rigid in the vertical direction.

The stabilizing structure may be comprised of any materials described in this section or elsewhere in this specification, including: flexible plastic such as silicone, polyurethane, rigid plastics such as polyvinyl chloride, semi-rigid plastics, semi-flexible plastics, biocompatible materials, composite materials, metals, and foam. In certain embodiments, the stabilizing structure may comprise a radio opaque material, to more readily allow a clinician to find pieces of the stabilizing structure within the wound.

As illustrated in Figure 2, the cells 8001 of the stabilizing structure may be staggered relative to each other. For example, the stabilizing structure may comprise adjacent rows of cells extending across the width of the stabilizing structure, wherein the cells of one row are not in line with cells of an adjacent row. Further details of the staggering of the cells and cell configurations are described below.

Some embodiments of stabilizing structure 8000 may have an outer perimeter that defines an at least partially elliptical shape when placed in the wound. In some embodiments, the stabilizing structure 8000 may have an outer perimeter that defines an at least partially elliptical shape when uncollapsed. In other embodiments, the outer perimeter may not be at least partially elliptical when uncollapsed, and may become elliptical when placed in the wound. In some embodiments, the stabilizing structure 8000 may have an outer perimeter that defines an at least partially rectangular shape when uncollapsed. In some embodiments, the outer perimeter is defined by the walls of the outermost cells. In other embodiments, the outer perimeter comprises a wound wall liner 8005 that extends along the height of the stabilizing structure, in addition to surrounding the walls defining outermost cells. The wound wall liner 8005 may partially or entirely surround the outer perimeter of the stabilizing structure. In some embodiments, the wound wall liner comprises hydrophobic material. In some embodiments, the wound wall liner comprises hydrophilic material.

In some embodiments, the stabilizing structure 8000 comprises a plurality of cells 8001 that are sized and configured to collapse inwardly (e.g., toward the central longitudinal axis of the stabilizing structure) with or without the application of negative pressure. This design may provide greater overall closure of the stabilizing structure 8000 to provide for maximum closure of the wound. The cells may be designed in a manner to facilitate closure of the stabilizing structure 8000 upon the application of negative pressure. In some embodiments, the stabilizing structure comprises cells that have a hexagon shape. In some embodiments, the stabilizing structure comprises cells that collapse along the width of the stabilizing structure while remaining relatively rigid along the length of the stabilizing structure and in the vertical direction. In some embodiments, the stabilizing structure comprises cells that have a concave-hexagon shape, such as a concave-hexagon shape wherein there are two internal angles greater than 180 degrees. In some embodiments, the stabilizing structure 8000 may contain size variations between cells located within a center portion and cells located within the longitudinal end portions of the stabilizing structure 8000. For example, cells at the longitudinal end portions may be larger or smaller than cells in the center portion.

In some embodiments, the stabilizing structure is configured to collapse by more than 50%, 60%, 70%, 80% or 90% in width upon application of negative pressure to the wound when the stabilizing structure is inserted into the wound.

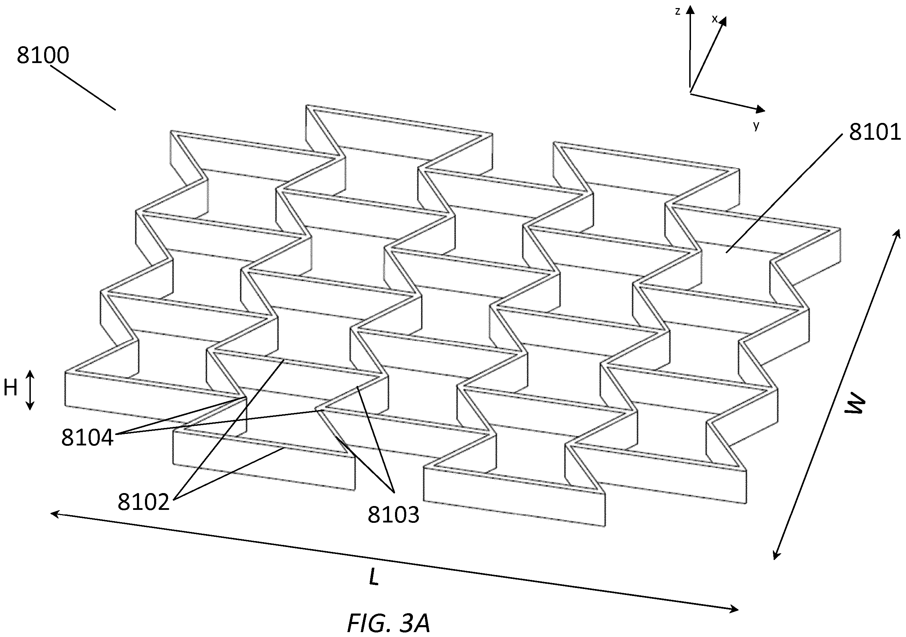

### Stabilizing Structures and Wound Closure Devices of Figures 3A-3B

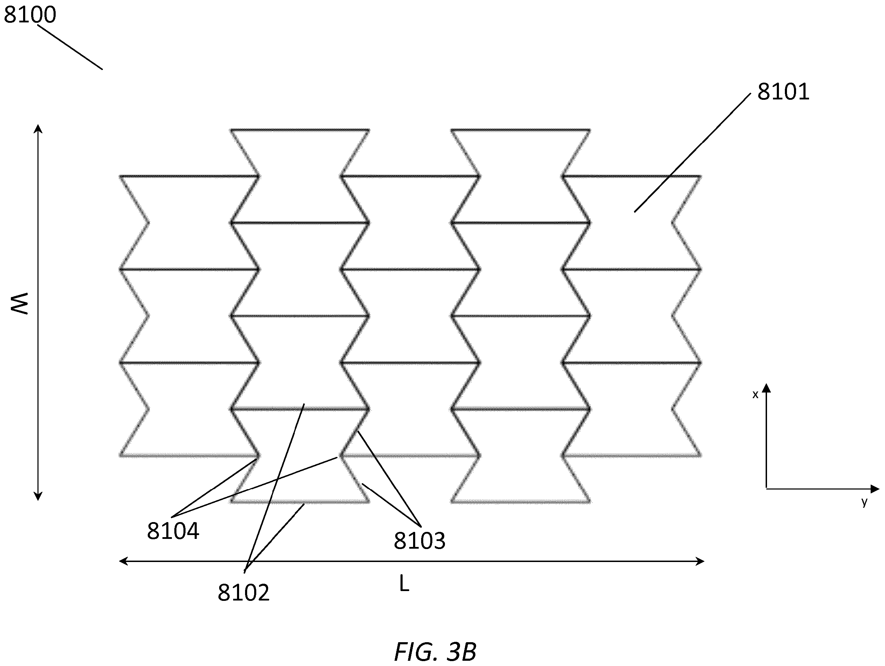

Figures 3A-3B illustrate an embodiment of a stabilizing structure 8100 which has similar configuration with the stabilizing structure 8000 described in relation to Figure 2. Figure 3A is a perspective view of the stabilizing structure 8100, and Figure 3B is a top view of the stabilizing structure 8100. In some embodiments, in a natural or uncollapsed state, the stabilizing structure 8100 comprises cells 8101 each defined by two straight, longitudinally-extending walls 8102 aligned in a parallel fashion along the length of the stabilizing structure and four side walls 8103 extending along the width of the stabilizing structure, from each end of two longitudinally-extending walls 8102 to nodes 8104, wherein two side walls 8103 meet at node 8104 to form an inner angle of greater than 180 degrees. In some embodiments, in the uncollapsed state, the stabilizing structure comprises cells having a concave-hexagon shape such as shown in Figures 3A-3B. Alternatively, in other embodiments, two side walls 8103 meet at node 8104 to form an inner angle of smaller than 180 degrees, so that the stabilizing structure comprises cells having a convex-hexagon shape. While the embodiments described herein in this section or elsewhere in this specification refer to uniformly sized concave-hexagon shaped cells shown by Figure 3 or uniformly sized hexagon shaped cells, it will be understood that the location, shape and relative sizes of the cells 8101 can be modified for any suitable embodiment and that their relative proportions can differ in various embodiments.

The cells 8101 of the stabilizing structure of Figures 3A-3B are staggered such that cells in one transverse row of cells are not aligned with cells in an adjacent transverse row of cells. For example, the stabilizing structure may comprise adjacent rows of cells extending across the width of the stabilizing structure, wherein the cells of one row are not in line with cells of an adjacent row. In some embodiments, the longitudinally-extending walls 8102 extend from a node 8104 where the side walls of another cell in the adjacent row meet, to another node where the side walls of another cell in another adjacent row on the other side meet, wherein the side walls 8103 of the cell meets at the node 8104 with the longitudinally-extending walls of another cells in the adjacent row. In some embodiments where the stabilizing structure comprises cells having a hexagon shape, a cell 8101 shares each of six walls with six adjacent cells, wherein the cell shares two longitudinally-extending walls with cells in a same row along the width and wherein the cell shares four side walls with cells in adjacent rows along the length. In some embodiments, the stabilizing structure comprises at least one node where three walls meet. In some embodiments, the stabilizing structure comprises at least one node where one longitudinally-extending wall and two side walls meet. In some embodiments, no more than three walls meet at any node in the stabilizing structure. In some embodiments, no more than one longitudinally-extending wall and no more than two side walls meet at any node in the stabilizing structure.

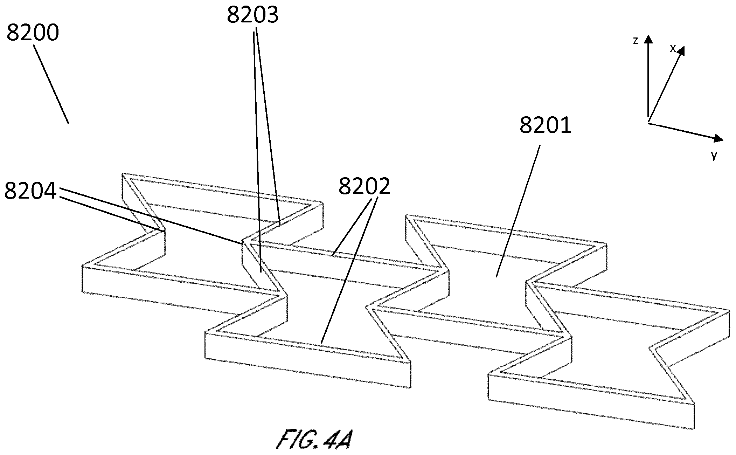

### Stabilizing Structures and Wound Closure Devices of Figures 4A-4D

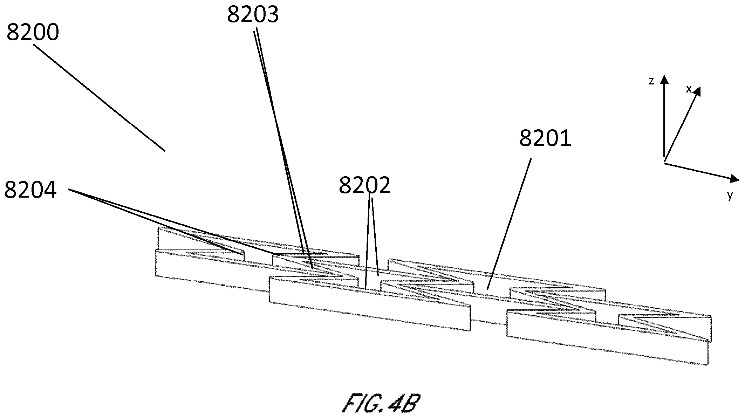

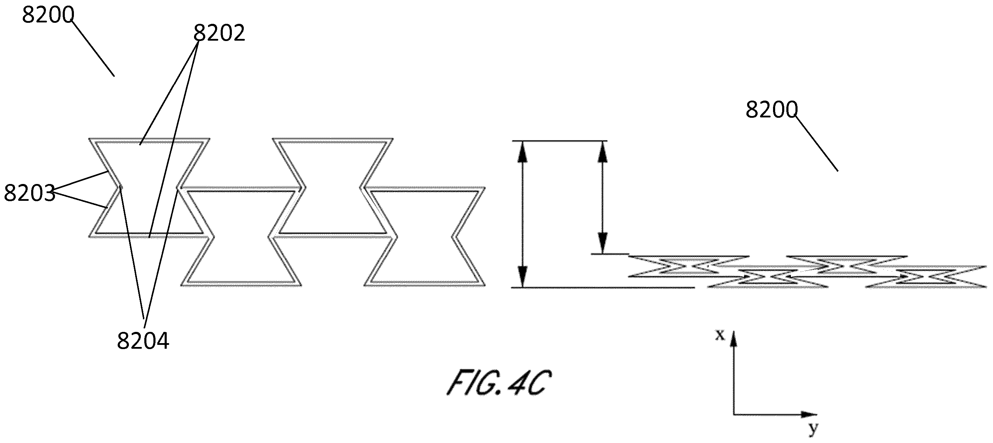

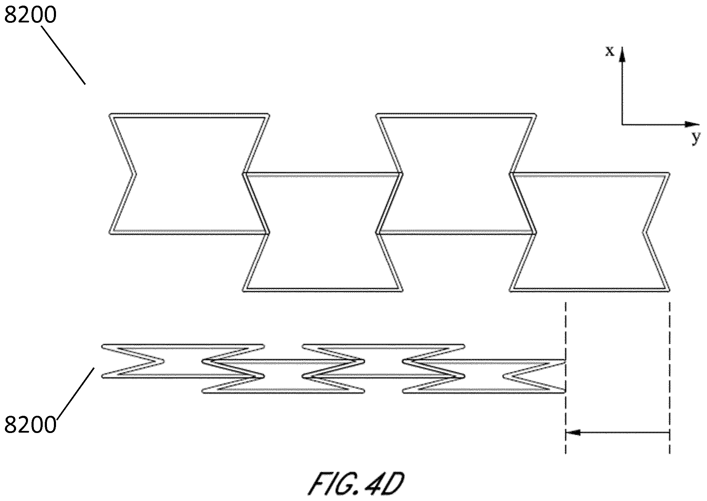

Figures 4A-4D are illustrations of an embodiment of a stabilizing structure 8200 which is similar to stabilizing structures as described above in relation to Figures 2-3B, or a portion thereof, wherein the cells have concave-hexagon shape. Figure 4A illustrates an embodiment of stabilizing structure 8200 in a natural, uncollapsed state. Figure 4B illustrates an embodiment of stabilizing structure 8200 in a collapsed state. Figure 4C illustrates a top view of an embodiment of the stabilizing structure 8200 before and after collapse, illustrating the amount that the width of the overall structure decreases. Figure 4D illustrates a top view of an embodiment of the stabilizing structure 8200 before and after collapse, illustrating how there is also a shortening of the overall length of the structure. As shown by Figures 4A-4D, in some embodiments, the stabilizing structure comprises cells that each collapse significantly along the width of the stabilizing structure while remaining relatively rigid along the length of the stabilizing structure and in the vertical direction. In some embodiments, the stabilizing structure comprises cells that collapse inwardly, so that cells do not increase dimension in any direction. In such embodiments, accordingly, the stabilizing structure does not increase overall dimension in any direction. In some embodiments, the stabilizing structure comprises cells that comprise longitudinal walls 8202 and side walls 8203, wherein longitudinal walls 8202 remain parallel with each other both in uncollapsed and collapsed configuration, while side walls 8203 form greater inner angles closer to 360 degrees in the collapsed configuration, as shown by Figures 4A-4D.

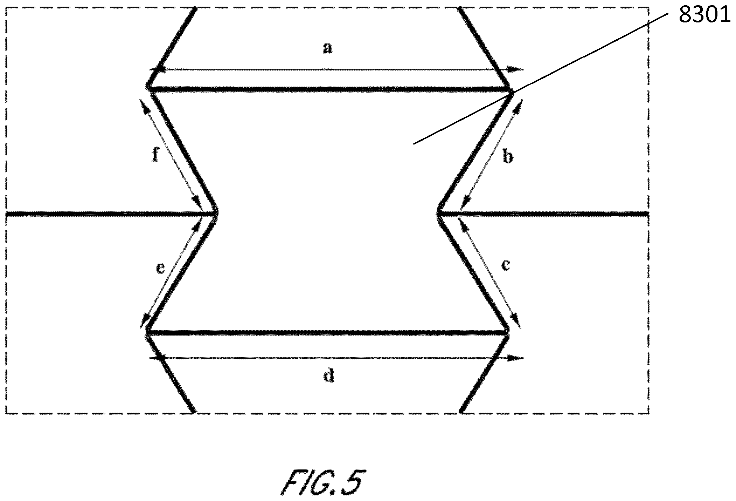

### Cell Configuration of Stabilizing Structures and Wound Closure Devices of Figure 5

The shape and length of each sides of cells of a stabilizing structure may be designed to facilitate maximum collapse of the stabilizing structure along its width. Figure 5 illustrates an embodiment of a cell 8301 of a stabilizing structure which has a similar configuration with stabilizing structures described above in relation to Figures 2-4D, wherein the cell has concave-hexagon shape. Among six walls of the cell 8301, two longitudinally-extending walls have lengths of a and d, respectively, while four side walls have lengths of b, c, e and f, respectively. In some embodiments, two longitudinally-extending walls have same length, so that a equals d. In some embodiments, two adjacent side wall may have same length, so that b equals c and e equals f. In some embodiments, four side walls have same lengths, so that b equals c, e and f.

To maximize collapse of the cell 8301, in some embodiments, the sum of lengths of longitudinal walls is equal to or greater than the sum of lengths of side walls. As shown in Figures 4A-4D, side walls and longitudinal walls may meet or gets very close when the stabilizing structure collapses. Turning back to Figure 5, if b+f is greater than a, two side walls having lengths of b and f may have to overlap when the cell collapses. Similarly, if c+e is greater than d, two side walls having lengths of c and e may have to overlap when the cell collapses. If opposing side walls of cells have to overlap with each other upon collapse, the width of each cell in collapsed state will be greater than cells which have opposing side which do not have to overlap with each other upon collapse. Accordingly, to maximize collapse of a stabilizing structure, a may be equal to or greater than b+f, and d may be equal to or greater than c+e. Thus, a+d may be equal or greater than b+c+e+f.

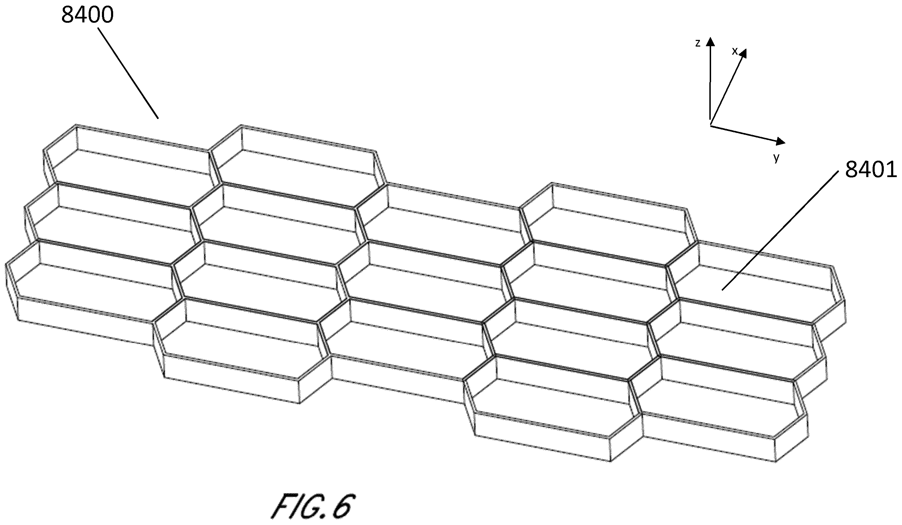

### Stabilizing Structures and Wound Closure Devices of Figures 6-7B

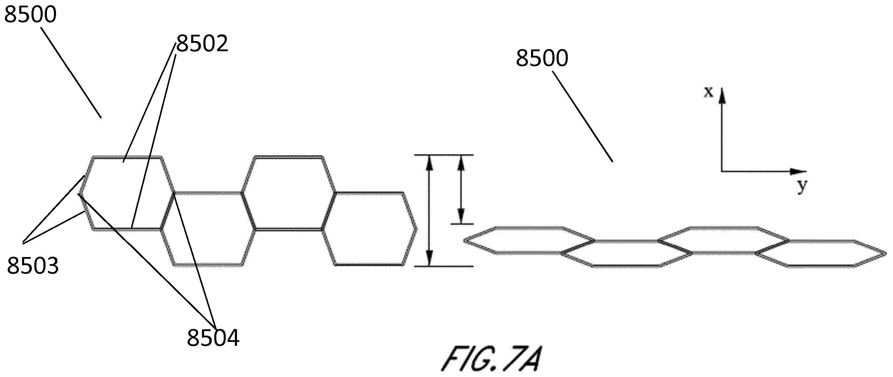

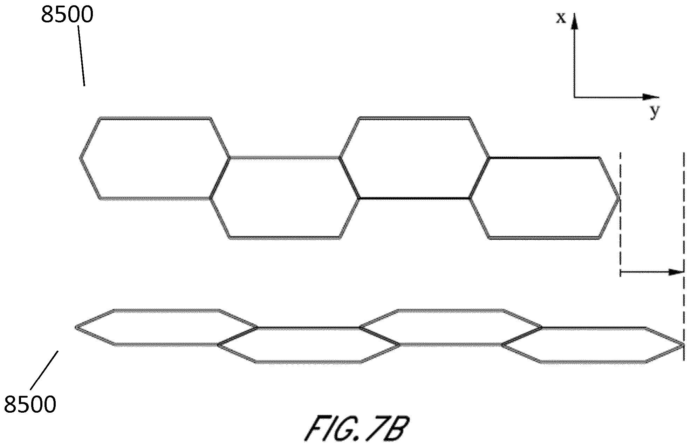

In other embodiments shown by Figure 6, the stabilizing structure 8400 comprises cells 8401 having a convex-hexagon shape wherein all interior angles are less than 180 degrees. Figures 7A and 7B are illustrations of an embodiment of a stabilizing structure 8500 which is similar to stabilizing structures 8400 of Figure 6 or a portion thereof, wherein the cells have convex-hexagon shape. In some embodiments, the convex-hexagon shape comprises longitudinal walls 8502 and side walls 8503, wherein longitudinal walls 8502 remain parallel with each other both in uncollapsed and collapsed configuration. The side walls 8503 in the uncollapsed configuration meet at nodes 8404 to form inner angles less than 180 degrees, and these side walls 8503 form smaller inner angles closer to 0 degrees in the collapsed configuration. As illustrated in Figure 7B, the stabilizing structure comprises cells that each collapse significantly along the width of the stabilizing structure while increasing in dimension along the length. Thus, a stabilizing structure comprising such cells may decrease in overall width when collapse, while increasing in overall length.

Cells of the stabilizing structure described herein may be designed and configured to promote uniform collapse of the stabilizing structure within both longitudinal end portions of the stabilizing structure and a central portion between the longitudinal end portions. For example, when a stabilizing structure as described herein is positioned within a wound and placed under negative pressure, the collapsed stabilizing structure may have a substantially uniform width along the entire length of the structure, such as shown in Figures 4A-4D and 7A-5B.

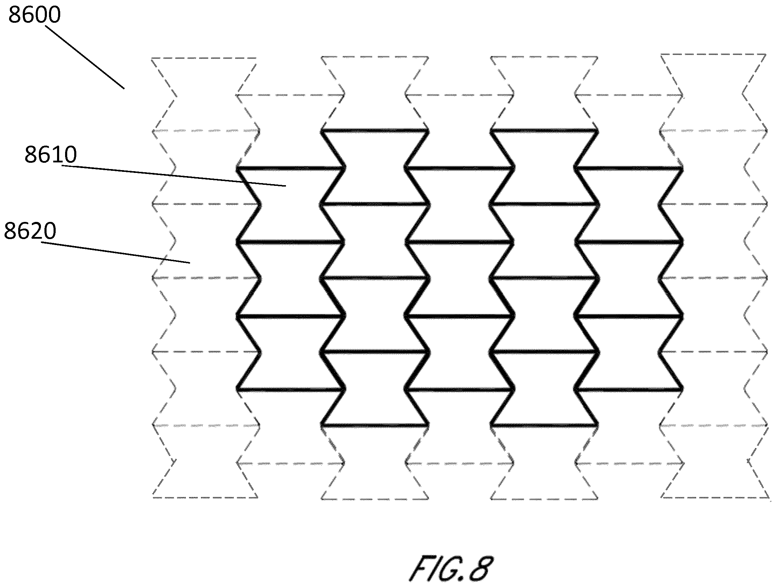

### Stabilizing Structures and Wound Closure Devices of Figures 8-9

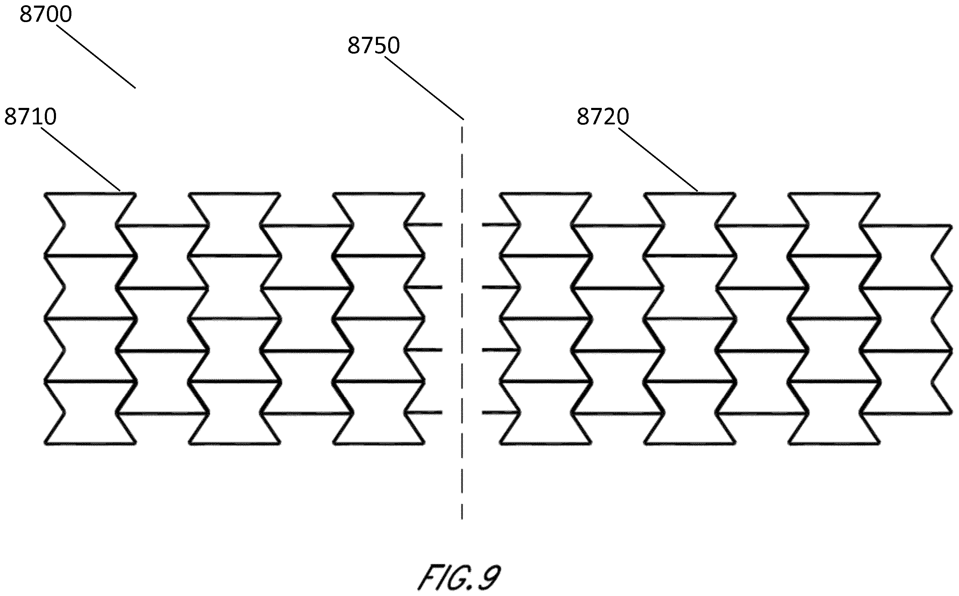

In some embodiments, the stabilizing structure may have an outer perimeter that defines an elliptical, generally elliptical, oval, generally oval, diamond, generally diamond or other shape in natural or uncollapsed state. These and other shapes may be created in some embodiments by fabricating or assembling a structure using a plurality of the cell shapes as described above, and in some embodiments the cell shapes and sizes are uniform for the entire structure. In some embodiments, as shown in Figure 8, the stabilizing structure 8600 may be configured to be cut or have separable or tearable portions 8620, such that the structure 8610 may be shaped into an elliptical, generally elliptical, oval, generally oval, diamond, generally diamond or other shape. Figure 8 illustrates how a generally rectangular stabilizing structure 8600 may be cut, separated or torn to form a generally oval or elliptical shape 8610 that may be suitable for placement into a wound. In some embodiments, the stabilizing structure is configured to be cut or have separable or tearable portions, such that the structure may be shaped to conform to a wound shape, such as an oval wound shape, whilst being uniform in cell geometry. In some embodiments, as shown in Figure 9, the stabilizing structure is configured to be cut or have separable or tearable portions, such that the structure may be sized into the desirable size. In some embodiments, the stabilizing structure 8700 can be configured to include perforations 8750 or detachable sections 8720 that allow portions of the device 8710 to separate from the remainder of the device. As described elsewhere in the specification, cuts or tears may be completed at any suitable location along the walls of the cells. In some embodiments, as shown in Figures 8-9, the stabilizing structure comprises cells with same size and shape, so that cuts or tears can be completed at any location along the walls of the cells.

## Claims

1. An apparatus for treating a wound with negative pressure wound therapy, comprising:
a stabilizing structure for insertion into a wound comprising:
a length corresponding to a y-direction and extending along a central longitudinal axis of the stabilizing structure between a first end and a second end of the stabilizing structure;
a width corresponding to an x-direction, the width being transverse to the length and extending along a central transverse axis of the stabilizing structure between a first side and a second side of the stabilizing structure; and
a height corresponding to a z-direction, the height being transverse to the length and the width and extending between a top surface and a bottom surface of the stabilizing structure;
wherein the length and width are each greater than the height;
wherein the stabilizing structure comprises a plurality of cells defined by one or more walls, the cells being provided side-by-side in a horizontal plane parallel to the x-direction and the y-direction, wherein each of the cells has a top end and a bottom end with an opening extending through the top and bottom ends in the z-direction;
wherein the plurality of cells are provided in a plurality of rows extending width-wise across the stabilizing structure, wherein cells of adjacent rows are staggered relative to each other; and
wherein the stabilizing structure is configured such that upon application of negative pressure to the wound when the stabilizing structure is inserted into the wound, the stabilizing structure collapses more in the horizontal plane than in the z-direction, and the stabilizing structure collapses more in the x-direction than in the y-direction; **characterized in that** at least one of the cells has a concave-hexagon shape comprising two parallel sides and two internal angles greater than 180 degrees.

2. The apparatus of claim 1, wherein at least one of the cells is defined by two straight walls aligned in parallel fashion along the y-direction and four side walls along the x-direction,
wherein side walls connect the two straight walls and
wherein at least two side walls form an inner angle greater than 180 degrees.

3. The apparatus of any one of the previous claims, wherein all of the cells have the concave-hexagon shape.

4. The apparatus of Claim 1 or Claim 3, wherein at least one cell having the concave-hexagon shape is surrounded by and shares a wall with six cells also having the concave-hexagon shape.

5. The apparatus of any one of Claims 2-4, wherein the sum of lengths of said two straight walls is equal to or greater than the sum of lengths of said four side walls.

6. The apparatus of any one of the previous claims, wherein the stabilizing structure comprises at least one node where three or fewer walls meet.

7. The apparatus of any one of the previous claims, wherein at all nodes three or fewer walls meet.

8. The apparatus of any one of the previous claims, wherein all of the cells of the stabilizing structure have the same shape and/or wherein all of the cells of the stabilizing structure have the same size.

9. The apparatus of any one of the previous claims, wherein cells are configured such that upon collapse, each cell shows zero or substantially no change in dimension in the y-direction.

10. The apparatus of any one of the previous claims, wherein the stabilizing structure is configured to allow portions of the structure to separate from a remainder of the structure and wherein the stabilizing structure comprises perforations or detachable sections that allow portions of the structure to separate from the remainder of the structure.

11. The apparatus of any of the previous claims, wherein the stabilizing structure further comprises a wound wall liner.

## Patentansprüche

1. Eine Vorrichtung zur Behandlung einer Wunde mit Unterdruckwundtherapie, die Folgendes beinhaltet:
eine stabilisierende Struktur zum Einführen in eine Wunde, die Folgendes beinhaltet:
eine Länge, die einer y-Richtung entspricht und sich entlang einer zentralen Längsachse der stabilisierenden Struktur zwischen einem ersten Ende und einem zweiten Ende der stabilisierenden Struktur erstreckt;
eine Breite, die einer x-Richtung entspricht, wobei die Breite quer zu der Länge ist und sich entlang einer zentralen Querachse der stabilisierenden Struktur zwischen einer ersten Seite und einer zweiten Seite der stabilisierenden Struktur erstreckt; und
eine Höhe, die einer z-Richtung entspricht, wobei die Höhe quer zu der Länge und der Breite ist und sich zwischen einer oberen Oberfläche und einer unteren Oberfläche der stabilisierenden Struktur erstreckt;
wobei die Länge und Breite jeweils größer als die Höhe sind;
wobei die stabilisierende Struktur eine Vielzahl von Zellen beinhaltet, die durch eine oder mehrere Wände definiert sind, wobei die Zellen in einer horizontalen Ebene parallel zu der x-Richtung und der y-Richtung nebeneinander bereitgestellt sind, wobei jede der Zellen ein oberes Ende und ein unteres Ende mit einer Öffnung, die sich durch das obere und untere Ende in der z-Richtung erstreckt, aufweist;
wobei die Vielzahl von Zellen in einer Vielzahl von Reihen, die sich der Breite nach über die stabilisierende Struktur erstrecken, bereitgestellt sind, wobei Zellen benachbarter Reihen relativ zueinander versetzt sind;
und
wobei die stabilisierende Struktur konfiguriert ist, sodass bei Anwendung von Unterdruck auf die Wunde, wenn die stabilisierende Struktur in die Wunde eingeführt wird, die stabilisierende Struktur mehr in der horizontalen Ebene als in der z-Richtung zusammengedrückt wird und die stabilisierende Struktur mehr in der x-Richtung als in der y-Richtung zusammengedrückt wird;
**dadurch gekennzeichnet, dass** mindestens eine der Zellen eine Form eines konkaven Sechsecks aufweist, die zwei parallele Seiten und zwei Innenwinkel beinhaltet, die größer als 180 Grad sind.

2. Vorrichtung gemäß Anspruch 1, wobei mindestens eine der Zellen durch zwei gerade Wände, die entlang der y-Richtung in paralleler Weise ausgerichtet sind, und vier Seitenwände entlang der x-Richtung definiert ist,
wobei die Seitenwände die zwei geraden Wände verbinden und
wobei mindestens zwei Seitenwände einen inneren Winkel bilden, der größer als 180 Grad ist.

3. Vorrichtung gemäß einem der vorhergehenden Ansprüche, wobei alle der Zellen die Form eines konkaven Sechsecks aufweisen.

4. Vorrichtung gemäß Anspruch 1 oder Anspruch 3, wobei mindestens eine Zelle, die die Form eines konkaven Sechsecks aufweist, von sechs Zellen, die auch die Form eines konkaven Sechsecks aufweisen, umgeben ist und mit ihnen eine Wand teilt.

5. Vorrichtung gemäß einem der Ansprüche 2-4, wobei die Summe von Längen der zwei geraden Wände gleich oder größer als die Summe der Längen der vier Seitenwände ist.

6. Vorrichtung gemäß einem der vorhergehenden Ansprüche, wobei die stabilisierende Struktur mindestens einen Knoten beinhaltet, wo sich drei oder weniger Wände treffen.

7. Vorrichtung gemäß einem der vorhergehenden Ansprüche, wobei sich an allen Knoten drei oder weniger Wände treffen.

8. Vorrichtung gemäß einem der vorhergehenden Ansprüche, wobei alle der Zellen der stabilisierenden Struktur dieselbe Form aufweisen und/oder wobei alle der Zellen der stabilisierenden Struktur dieselbe Größe aufweisen.

9. Vorrichtung gemäß einem der vorhergehenden Ansprüche, wobei Zellen konfiguriert sind, sodass beim Zusammendrücken jede Zelle null oder im Wesentlichen keine Größenveränderung in der y-Richtung zeigt.

10. Vorrichtung gemäß einem der vorhergehenden Ansprüche, wobei die stabilisierende Struktur konfiguriert ist, um Teilen der Struktur zu erlauben, sich von einem Rest der Struktur zu trennen, und wobei die stabilisierende Struktur Perforationen oder abnehmbare Abschnitte beinhaltet, die Teilen der Struktur erlauben, sich von dem Rest der Struktur zu trennen.

11. Vorrichtung gemäß einem der vorhergehenden Ansprüche, wobei die stabilisierende Struktur ferner eine Wundwandbekleidung beinhaltet.

## Revendications

1. Un appareil pour le traitement d'une plaie avec une thérapie des plaies par pression négative, comprenant :
une structure de stabilisation pour insertion dans une plaie comprenant :
une longueur correspondant à une direction y et s'étendant suivant un axe longitudinal central de la structure de stabilisation entre une première extrémité et une deuxième extrémité de la structure de stabilisation ;
une largeur correspondant à une direction x, la largeur étant transversale à la longueur et s'étendant suivant un axe transversal central de la structure de stabilisation entre un premier côté et un deuxième côté de la structure de stabilisation ; et
une hauteur correspondant à une direction z, la hauteur étant transversale à la longueur et à la largeur et s'étendant entre une surface de dessus et une surface de dessous de la structure de stabilisation ;
où la longueur et la largeur sont chacune supérieures à la hauteur ;
où la structure de stabilisation comprend une pluralité d'alvéoles définies par une ou plusieurs parois, les alvéoles étant fournies côte à côte dans un plan horizontal parallèle à la direction x et à la direction y, où les alvéoles présentent chacune une extrémité de dessus et une extrémité de dessous avec une ouverture s'étendant à travers les extrémités de dessus et de dessous dans la direction z ;
où la pluralité d'alvéoles sont fournies en une pluralité de rangées s'étendant dans le sens de la largeur d'un côté à l'autre de la structure de stabilisation, où des alvéoles de rangées adjacentes sont décalées les unes par rapport aux autres ;
et
où la structure de stabilisation est configurée de telle sorte que suite à l'application d'une pression négative sur la plaie lorsque la structure de stabilisation est insérée dans la plaie, la structure de stabilisation s'affaisse plus dans le plan horizontal que dans la direction z, et la structure de stabilisation s'affaisse plus dans la direction x que dans la direction y ;
**caractérisé en ce qu'**au moins une des alvéoles présente une forme en hexagone concave comprenant deux côtés parallèles et deux angles internes supérieurs à 180 degrés.

2. L'appareil de la revendication 1, où au moins une des alvéoles est définie par deux parois droites alignées de façon parallèle suivant la direction y et quatre parois de côté suivant la direction x,
où des parois de côté se raccordent aux deux parois droites et
où au moins deux parois de côté forment un angle interne supérieur à 180 degrés.

3. L'appareil de n'importe laquelle des revendications précédentes, où les alvéoles présentent toutes la forme en hexagone concave.

4. L'appareil de la revendication 1 ou de la revendication 3, où au moins une alvéole présentant la forme en hexagone concave est entourée par et partage une paroi avec six alvéoles présentant aussi la forme en hexagone concave.

5. L'appareil de n'importe laquelle des revendications 2 à 4, où la somme de longueurs desdites deux parois droites est égale ou supérieure à la somme de longueurs desdites quatre parois de côté.

6. L'appareil de n'importe laquelle des revendications précédentes, où la structure de stabilisation comprend au moins un nœud où trois parois ou moins se rejoignent.

7. L'appareil de n'importe laquelle des revendications précédentes où, à tous les nœuds, trois parois ou moins se rejoignent.

8. L'appareil de n'importe laquelle des revendications précédentes, où les alvéoles de la structure de stabilisation présentent toutes la même forme et/ou où les alvéoles de la structure de stabilisation présentent toutes la même taille.

9. L'appareil de n'importe laquelle des revendications précédentes, où des alvéoles sont configurées de telle sorte que suite à l'affaissement, chaque alvéole ne montre aucun ou substantiellement pas de changement de dimension dans la direction y.

10. L'appareil de n'importe laquelle des revendications précédentes, où la structure de stabilisation est configurée afin de permettre à des portions de la structure de se séparer d'un reste de la structure et où la structure de stabilisation comprend des perforations ou des sections détachables qui permettent à des portions de la structure de se séparer du reste de la structure.

11. L'appareil de n'importe lesquelles des revendications précédentes, où la structure de stabilisation comprend en sus un chemisage de paroi de plaie.
